# EUROPEAN PATENT APPLICATION

(11) **EP 2 799 072 A1**
(43) Date of publication of application: **05.11.2014**
(21) Application number: 12861663.8
(22) Date of filing: 05.10.2012
(51) Int. Cl.: A61K 31/4709, A61K 47/26, A61K 47/02, A61K 9/28, A61K 9/16, A61P 31/04

(54) **SOLID PHARMACEUTICAL COMPOSITION COMPRISING AN ANTIBIOTIC FROM THE QUINOLONE FAMILY AND METHOD FOR THE PRODUCTION THEREOF**

(30) Priority: 26.12.2011 BR PI1106900
(71) Applicant: EMS S.A., 13186-901 Sao Paulo - SP (BR)
(72) Inventor: CATELLI, Etamyr Eduardo Ribeiro, 13186-901 São Paulo - SP (BR); ROQUE, Samira Eloá de Paula, 13186-901 São Paulo - SP (BR); MARQUES, Ricardo Vian, 13186-901 São Paulo - SP (BR); COVESI, Leticia Khater, 13186-901 São Paulo - SP (BR)
(74) Representative: Cabinet Plasseraud
(86) International application number: PCT/BR2012/000383
(87) International publication number: WO 2013/097003

(57) **Abstract**

The present invention aims to provide a solid pharmaceutical composition comprising: (a) an effective antibacterial quantity of antibiotic from the quinolone family, preferably, moxifloxacin or a pharmaceutically acceptable salt thereof; and (b) a pharmacologically acceptable carrier or excipient compatible with the active ingredient, said excipient being lactose-free.

The invention also includes a process for obtaining a solid pharmaceutical composition comprising, as an active ingredient, an antibiotic from the quinolone family, said process comprising the steps of: (a) mixing and homogenizing the active ingredient and dry excipients in the granulator, i.e., at least one diluent and at least one disintegrant; (b) dissolving at least one binder in an organic solvent selected from the group consisting of isopropyl alcohol, acetone, ethanol, dichloromethane or mixtures thereof; (c) granulating the dried mixture of step (a) with the solution of step (b); (d) sorting and drying the granules of step (c) at a suitable temperature; (e) sorting the dried granulate; (f) mixing and homogenizing the granules of step (e) with an additional amount of disintegrant to obtain the composition of the invention.

## Description

### Field of the Invention

The present invention relates to a pharmaceutical composition for the treatment of human and veterinary bacterial infections, said composition containing, as an active ingredient, an antibiotic from the quinolone family and excipients having enhanced stability, especially with regard to undesirable properties caused by hygroscopicity and/or by the absorption of water during storage (shelf life) and during pharmaceutical processing. The invention also covers the process for obtaining said pharmaceutical composition. Preferably, the said antibiotic of the quinolone family is moxifloxacin.

### Fundamentals of the Invention

The class of quinolones is characterized by the inhibition of the bacterial enzyme DNA topoisomerase II (DNA gyrase) and topoisomerase IV. The class of quinolones, started in 1962 by Lepper, had a very large boost from the introduction of ciprofloxacin in the late 1980s, which was the first quinolone available for systemic use, in view of its pharmacokinetic behavior and the expansion of its spectrum of action. A most striking breakthrough came in 1997 with the introduction of levofloxacin, the first quinolone considered third generation and that, beyond the improvements previously obtained with ciprofloxacin, still has a high pneumococcal activity, which places it as the drug of choice or alternative therapy for infections of the upper and lower respiratory tract in various situations. In this class, the new fluoroquinolones such as gatifloxacin, moxifloxacin, gemifloxacin, and among others, are available.

Moxifloxacin, also known as CDCH, was disclosed in document EP0350733 (corresponding to patents US4990517 and US5607942) published on 01-17-1990. The molecule was registered under CAS number 151096-09-2, being named 1-cyclopropyl-6-fluoro-1,4-dihydro-8-methoxy-7-[(4αS,7αS)-octahydro-6H-pyrrolo[3,4-β]pyridin-6-yl]-4-oxo-3-quinoline carboxylic acid (IUPAC nomenclature). The chemical formula of this compound is shown below. 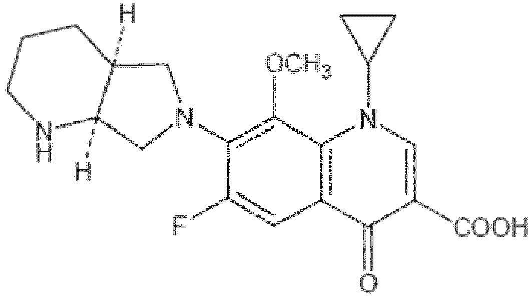 The synthesis of moxifloxacin and process improvements are described in documents EP0350733, EP0757990 and EP0550903.

In patent PI9605968 (corresponding patents US5849752 and EP0780390) the pharmaceutical compositions comprising moxifloxacin hydrochloride monohydrate are described. According to this document, the crystalline monohydrate improves control of dosage of the active ingredient and the quality and stability of liquid pharmaceutical preparations. Additionally, crystals of different shapes are obtained from the needles, characteristic of the anhydrous form, which hinder the flow of moxifloxacin during the preparation of solid forms.

In patent PI9915208 (corresponding patents US6610327 and EP1128831) a pharmaceutical preparation of moxifloxacin is described in which the presence of lactose, added at a concentration in the range of 2.5 to 25%, enabled the improvement of the tensile strength and hardness of the tablet. However, it is known that polymorphic forms of the same drug may have substantial differences in pharmaceutical properties, such as dissolution profile, bioavailability and stability of the molecule. Moreover, polymorphic forms may have different particle size and hardness, among other properties. Furthermore, it is well known that a significant number of individuals have an intolerance to lactose.

Another attempt at improving the stability and processability of moxifloxacin during and after the preparation of the pharmaceutical formulation, while maintaining, and even increasing considerably the quantity of lactose as an essential excipient, is described in document WO2011086577. In this formulation, the quantity of lactose is situated in a range between 20 and 50%, and preparation of the composition includes a granulation step using water.

An attempt to improve the properties of pharmaceutical compositions of moxifloxacin is disclosed in WO2005020998. In this proposal, the solution to the problem of the prior art is based on providing at least one intragranular and extragranular excipient substantially insoluble in water. In the preferred embodiment of the invention described in WO2005020998, the substantially water-insoluble excipient has solubility less than lactose and is selected from the group comprising microcrystalline cellulose, pregelatinized starch, corn starch and dibasic calcium phosphate dihydrate. The process for preparing the pharmaceutical composition described herein also involves a granulation step with water.

The anhydrous form of moxifloxacin hydrochloride is more suitable than monohydrate for obtaining a soluble pharmaceutical formulation. However, since moxifloxacin is a hygroscopic compound, hydration is common both in storage and during pharmacotechnical processing when water is used in the wet granulation step. It is for this reason that the monohydrate form of moxifloxacin is the preferred choice to avoid physical instability of pharmaceutical preparations.

In this sense, a pharmaceutical composition of moxifloxacin is highly desirable, employing the anhydrous form of the active ingredient, which is stable in storage and during pharmacotechnical operations, including granulation. It is also highly desirable that said pharmaceutical composition does not include lactose among the excipients, which can cause problems of intolerance in individuals who need this medicine. In other words, there is a need for stable formulations containing CDCH to prevent degradation from moisture and heat caused during the production process, and also during storage of the product, thereby providing good conditions for manufacturing of conventional oral pharmaceutical forms as tablets or hard gelatin capsules, or equivalent solid forms.

### Summary of the Invention

The main objective of the present invention is to achieve stable solid pharmaceutical compositions of CDCH with adequate release to achieve the desired therapeutic effect.

A first aspect of the present invention relates to a pharmaceutical composition of CDCH in the anhydrous form in which stability was surprisingly achieved in the absence of lactose.

A second aspect of the invention relates to a process for preparing a stable pharmaceutical composition of the invention, wherein the granulation step is carried out without the use of water.

A third aspect of the present invention is to produce a solid form which is pharmaceutically equivalent to the drug marketed under the name Avalox ®.

The first embodiment of the invention relates to a solid pharmaceutical composition comprising: (a) an effective antibacterial quantity of antibiotic from the quinolone family, preferably moxifloxacin or a pharmaceutically acceptable salt thereof; and (b) a vehicle or excipients pharmacologically acceptable and compatible with the active ingredient, said composition being preferably free of lactose. The carrier or excipients include at least one diluent, at least one binder and at least one disintegrant. Preferably, the composition comprises: anhydrous moxifloxacin hydrochloride; two diluents selected from microcrystalline cellulose and/or mannitol; crosslinked polyvinylpyrrolidone as a binder (povidone); as disintegrant, cross-linked sodium carboxymethyl cellulose (croscarmellose sodium); and a lubricant such as magnesium stearate. The solid dosage forms containing the composition of the invention include tablets, optionally coated, capsules, granules, lozenges and the like. More preferably, the composition of the invention is in the form of coated tablets, wherein the coating comprises: (i) at least one polymer coating, (ii) at least one plasticizer, (iii) at least one dye and (iv) at least one solvent. Even more preferably, the polymer coating is hydroxypropyl methylcellulose (hypromellose) and the plasticizer is polyethylene glycol (macrogol); the dye is red iron oxide and/or titanium dioxide; and the solvent is isopropyl alcohol and/or methylene chloride.

The second embodiment of the present invention relates to a process for obtaining a solid pharmaceutical composition comprising, as the main active ingredient, an antibiotic of the quinolone family, with said process comprising the steps of: (a) mixing and homogenizing the active ingredient and the dry excipients in a granulator, i.e., at least one diluent and at least one disintegrant; (b) dissolving at least one binder in an organic solvent selected from the group consisting of isopropyl alcohol, acetone, ethanol, dichloromethane or mixtures thereof; (c) granulating the dried mixture of step (a) with the solution of step (b); (d) classifying and drying, at a suitable temperature, the wet granulation from step (c) to an appropriate level; (e) classifying the dried granulate; (f) mixing and homogenizing the granules of step (e) with an additional quantity of disintegrant to obtain the composition of the invention in the form of granules.

Preferably, the pharmaceutical composition of the invention is in the form of coated tablets. Therefore, in addition to steps (a) to (f) of the process of the invention further includes the steps of: (g) compressing the dried granulated mixture of step (f); and (h) preparing a coating mixture and coating the tablet obtained in step (g) to obtain a coated tablet.

Also preferably, the pharmaceutical composition of the invention is in the form of capsules. Therefore, in addition to steps (a) to (f) the process of the invention further includes the step of: (i) encapsulating, in hard gelatin capsules, a granulated dried mixture from step (f) to obtain capsules.

### Brief Description of the Drawings

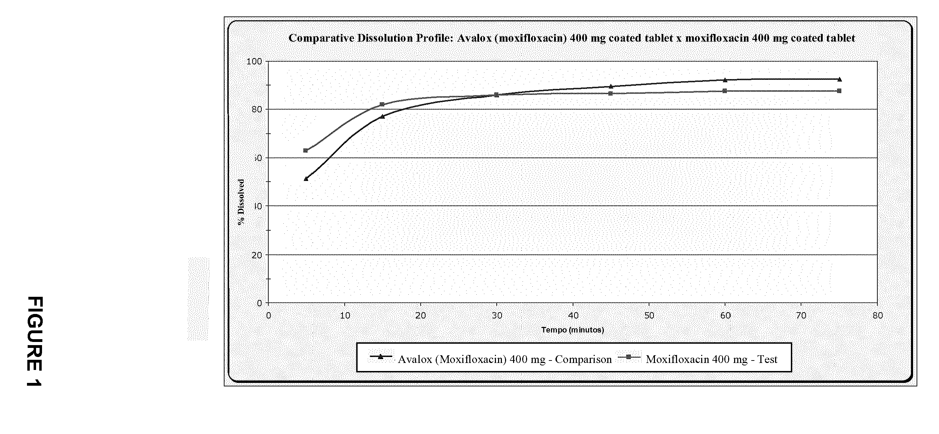

Figure 1 shows a comparison between the dissolution profiles of the pharmaceutical composition of the present invention, in the form of a coated tablet containing 400 mg of anhydrous moxifloxacin with a coated tablet of the reference product Avalox ®.

### Detailed Description of the Invention

The present invention deals with obtaining a solid pharmaceutical composition comprising an antibiotic of the quinolone class and excipients that provide stability of said composition without adding lactose.

Preferably, the antibiotic of the quinolone class is moxifloxacin or a pharmaceutically acceptable salt thereof. More preferably, the antibiotic of the quinolone family is moxifloxacin hydrochloride. Even more preferably, the moxifloxacin hydrochloride used in the pharmaceutical composition of the invention is in anhydrous form.

The excipients used in the composition of the invention are selected from the classes of diluents, binders, disintegrants and lubricants. In particular, the flow regulating excipients are employed to improve the disintegration characteristics, the flavor, and to reduce the tendency for adhesion of the mixture in the compression machine, improving the process of compression.

The diluent employed in the composition of the invention is selected from the group consisting of: microcrystalline cellulose, mannitol, pregelatinized starch, anhydrous calcium phosphate, di-or tribasic anhydrous calcium phosphate or monohydrate. Preferably, two solvents are employed in the composition of the present invention, more preferably mannitol and microcrystalline cellulose.

The disintegrant used in the composition of the invention is selected from the group consisting of: croscarmellose sodium, sodium starch glycolate, crospovidone, low-substituted hyprolose and pregelatinized starch. Preferably, the disintegrant used in the composition of the invention is croscarmellose sodium.

The binder used in the composition of the invention is selected from the group consisting of povidone, copovidone, hypromellose, hyprolose, starch and pregelatinized starch. Preferably, the binder used in the composition of the invention is povidone.

The lubricant used in the composition of the present invention is selected from the group consisting of: stearic acid and its metal salts, sodium stearyl fumarate, glyceryl behenate, talc, and macrogol.

Preferably, the lubricant is magnesium stearate.

In the particularly preferred embodiment, the solid pharmaceutical composition of the invention comprises: (a) anhydrous moxifloxacin hydrochloride and (b) the excipients comprising (i) mannitol and microcrystalline cellulose as diluents; (ii) croscarmellose sodium as a disintegrant; (iii) povidone as binder; and (iv) magnesium stearate as a lubricant.

The pharmaceutical composition of the present invention comprises: (i) 20-70% of active ingredient, (ii) 2 to 10% binder, (iii) 30-60% diluent, (iv) 1-10% disintegrant, and (v) 0.1-5% lubricant. The active ingredient is any antibiotic of the quinolone family, which includes norfloxacin, ciprofloxacin, ofloxacin, levofloxacin and moxifloxacin. Preferably, the active ingredient of the composition of the invention is moxifloxacin, and more preferably is moxifloxacin hydrochloride.

In a preferred embodiment of the invention, the composition comprises: 40-60% moxifloxacin, 15-25% microcrystalline cellulose, 5-15% mannitol, 0.5-1.5% of magnesium stearate, 3-6% croscarmellose sodium and 2-4% povidone. In a more preferred embodiment of the invention, the composition comprises: 50-60% anhydrous moxifloxacin hydrochloride, 18-23% microcrystalline cellulose, 11-12% mannitol, 1.0-1.5% of magnesium stearate, 4-5% croscarmellose sodium, and 2.5-3.5% povidone. The percentages are related to weight/weight.

Fundamentally, the composition of the present invention is in the form of granules obtained by granulation with an organic solvent and without the presence of water.

Thus, the pharmaceutical form of the composition of the invention may be granular. Alternatively, the granules may be placed in capsules, preferably made of gelatin. The granules may also be compressed to obtain tablets.

In preferred form, the tablets of the invention are coated to enhance the stability of the active ingredient. Alternatively, the coating may confer modified release characteristics of the active ingredient, for example, delayed release, controlled release. The coating consists of one or more suitable polymers, plasticizers, colorants/opacifiers, solvents/ vehicle, flavorings, sweeteners, surfactants, antioxidants, antimicrobials/preservatives. The film-forming polymer may be selected from the group consisting of: hydroxypropyl cellulose, methyl cellulose, polyvinylpyrrolidone and pharmaceutically acceptable acrylic polymers. The plasticizer may be selected from the group consisting of: polyethylene glycol, propylene glycol, and triacetin. The colorant/opacifier may be selected from the group consisting of: red iron oxide, yellow iron oxide, titanium dioxide and talc. The solvent should be a pharmaceutically acceptable organic solvent to avoid hydration of the active ingredient. Solvents suitable for the coating are low molecular weight alcohols, preferably isopropyl alcohol and chlorinated organic compounds, for example, methylene chloride.

The present invention also relates to the process for obtaining a solid pharmaceutical composition of the present invention. In general, the pharmaceutical composition of the invention can be produced as follows: (a) adding the active ingredient and excipients to the granulator; (b) granulating the mixture with a solvent after drying and; (c) adding additional disintegrant and lubricant to the dry granulate until obtaining a homogeneous mixture; and (d) bringing the final mixture to compression or encapsulation.

Surprisingly, it was found that the tablets or capsules of CDCH exhibit excellent stability, optimal compression and adequate release without the use of lactose. Besides enabling stable compositions of anhydrous CDCH, the process of the invention also minimizes the effects of adhesion and cracking phenomena often encountered during the preparation and manipulation of CDCH, thereby providing easy manipulation of tablets by direct compression and/or granulation or encapsulation.

These advantages are achieved due to the fact that organic solvent is employed in the granulation step. In other words, the CDCH is not exposed to water during the entire process, due to the fact that it is conducted in the absence of water. An additional advantage is that the granules produced by the process of the present invention substantially reduce the phenomena of adhesion and attain the proper fluidity, making the mixture ideal for formulation into tablets or hard gelatin capsules.

The following embodiments of the invention are presented by way of example. However, it should be understood that such examples are provided for illustrative purposes only, and that various modifications or changes, in light of the embodiments disclosed herein, will be suggestive to specialists in the art and must be included within the spirit and scope of this disclosure and the scope of the accompanying claims.

### EXAMPLES

### EXAMPLE 1: Obtaining coated tablets of CDCH

The quantity of ingredients of the CDCH composition of the invention in the form of coated tablets are shown in Table 1.

**Table 1: Coated tablets of CDCH**

| **Raw Material** | **Quantity (%)** | **Function** |
|---|---|---|
| **Core:** | | |
| moxifloxacin hydrochloride | 55.9% | Active substance |
| microcrystalline cellulose | 20.3% | Diluent |
| Manitol | 11.2% | Diluent |
| croscarmellose sodium | 4.8% | Disintegrant |
| Povidone | 2.9% | Binder |
| magnesium stearate | 1.0% | Lubricant |
| isopropyl alcohol | N/A * | Solvent |

| **Coating:** | | |
|---|---|---|
| hipromelose +polyethylene glycol | 2.9% | Polymer coating |
| red iron oxide | 0.1% | Coloring |
| titanium dioxide | 0.9% | Coloring |
| isopropyl alcohol | N/A* | Solvent |
| methylene chloride | N/A* | Solvent |

| | | |
|---|---|---|
| N/A* indicates that the compound occurred during the evaporation process | | |

### Manufacturing Procedure:

The process according to the invention comprises the following steps:

### A. Obtaining granular and tablet core of CDCH

1. Add to the Granulator the following raw materials: moxifloxacin hydrochloride, microcrystalline cellulose, mannitol and croscarmellose sodium (half the amount); and homogenize the dry mixture.
2. In a suitable container, dissolve the povidone in isopropyl alcohol.
3. Granulate the powdered mixture obtained in step 1, with the solution prepared in step 2.
4. Gradually add isopropyl alcohol to the Granulator until reaching the ideal point of granulation.
5. Sort the granulate in a perforated mesh.
6. Dry the granulate in an oven.
7. Sort the dry granulate in a perforated mesh and add to the Blender.
8. Transfer the remaining croscarmellose sodium to the Blender and homogenize.
9. Sort the magnesium stearate in a perforated mesh, transfer to the Blender and homogenize.
10. Proceed to compressing the powdered mixture as described in the prior art.

### B. Coated tablet of CDCH

1. In a suitable container, add, under agitation, the following raw materials: isopropyl alcohol, methylene chloride, hypromellose + macrogol; and homogenize the mixture.
2. In another suitable container, with the aid of a blender, disperse the following raw materials: isopropyl alcohol, titanium dioxide and red iron oxide.
3. Rinse the Blender with isopropyl alcohol and add the solution obtained in step 1 and the dispersion obtained in step 2; blend the mixture under agitation.
4. Coat and package the tablets.

### EXAMPLE 2: Obtaining capsules of CDCH

The quantity of ingredients of the composition of CDCH of the invention in capsule form is described in Table 2.

**Table 2: Capsules of CDCH**

| **Raw Material** | **Quantity (%)** | **Function** |
|---|---|---|
| moxifloxacin hydrochloride | 58.2% | Active substance |
| microcrystalline cellulose | 21.1% | Diluent |
| Manitol | 11.7% | Diluent |
| croscarmelose sodium | 5.0% | Disintegrant |
| Povidone | 3.0% | Binder |
| magnesium stearate | 1.0% | Lubricant |
| isopropyl alcohol | N/A* | Solvent |

| | | |
|---|---|---|
| N/A* indicates that the compound occurred during the evaporation process | | |

### Manufacturing Process:

The process according to the invention comprises the following steps:
1. Add the following raw materials to the Granulator: moxifloxacin hydrochloride, microcrystalline cellulose, mannitol and croscarmellose sodium (half the amount); and blend the dry mixture.
2. In a suitable container, dissolve the povidone in isopropyl alcohol.
3. Granulate the powdered mixture obtained in step 1, with the solution prepared in step 2.
4. Gradually add isopropyl alcohol to the Blender until reaching the ideal point of granulation.
5. Sort the moist granules in a perforated mesh.
6. Dry the granulate in an oven.
7. Sort the dry granulate in a perforated mesh and add to the Blender.
8. Transfer the remaining croscarmellose sodium to the Blender and homogenize.
9. Sort the magnesium stearate in a perforated mesh, transfer to the Blender and homogenize.
10. Proceed to the encapsulation of the powdered mixture.

### EXAMPLE 3: Stability Study of CDCH of the tablet obtained in Example 1

The coated tablets of CDCH obtained in Example 1 were subjected to an accelerated stability test. The conditions of this test were as follows: (i) temperature of 40 +/- 2° C and (ii) a relative humidity of 75 +/- 5%. The amount of intact drug, which is a characteristic that proves the stability of the drug, was assessed after 90 and 180 days. The results in improved stability of the composition of anhydrous moxifloxacin hydrochloride are shown in Table 3.

**Table 3: Stability Test of Tablets Coated in CDCH**

| **TEST** | **INICIAL** | **90 DIAS** | **180 DIAS** |
|---|---|---|---|
| Moxifloxacin content (%) | 100 | 99 | 98 |
| Hardness | 15.8 Kp | 15.4 Kp | 15.5 Kp |

### EXAMPLE 4: Dissolution Profile Comparison

The CDCH coated tablets of 400 mg obtained in Example 1, were compared to the reference product Avalox ® 400 mg as to the dissolution profile.

The following test conditions were employed:
Dissolution medium: Hydrochloric acid 0.1N pH 1.2
Tank volume: 900 ml
Device II (paddles)
Rotation: 50 rpm
Collection times: 5, 15, 30, 45, 60 and 75 minutes
Bath temperature: 37° C

The result is shown in Figure 1, where F1 = 3.33 and F2 = 56.86. It is evident that the dissolution profile of the tablet of the present invention and the Avalox® product are equivalent.

All publications and patent applications mentioned in this specification are indicative of the level of those skilled in the art to which the invention relates. All publications and patent applications are incorporated herein by reference to the same extent as if each individual publication or patent application were each specifically and individually indicated to be incorporated for ease of reference.

Although certain embodiments have been described, they were presented only as a means of illustration, and are not intended to limit the scope of the invention. In fact, new embodiments described herein can be implemented in a variety of other forms, more than that, various omissions, substitutions and changes in the way in which the embodiments are described herein can be made without departing from the spirit of the inventions. The accompanying claims of this description and their equivalents are considered to cover such forms or modifications as they may be within the scope and spirit of the inventions.

## Claims

1. A solid pharmaceutical composition including an antibiotic from the quinolone family comprising:
(a) an effective antibacterial quantity of said antibiotic from the quinolone family or a pharmaceutically acceptable salt thereof;
(b) a pharmaceutically acceptable excipient compatible with the active ingredient,
said composition being free of lactose.

2. A composition according to claim 1, wherein the antibiotic from the quinolone family is selected from the group consisting of norfloxacin, ciprofloxacin, ofloxacin, levofloxacin and moxifloxacin.

3. A composition according to claim 2, wherein the antibiotic from the quinolone family is moxifloxacin.

4. A composition according to claim 3, wherein moxifloxacin is in anhydrous form.

5. A composition according to claim 4, wherein the anhydrous moxifloxacin is in the form of hydrochloride salt.

6. A composition according to claim 1, wherein the excipient comprises (i) at least one diluent, (ii) at least one disintegrant, (iii) at least one binder and (iv) at least one lubricant.

7. A composition according to claim 6, wherein at least one diluent is selected from the group consisting of microcrystalline cellulose, mannitol, pregelatinized starch, anhydrous calcium phosphate, di-or tribasic anhydrous calcium phosphate or monohydrate.

8. A composition according to claim 6, wherein at least one disintegrant is selected from the group consisting of croscarmellose sodium, sodium starch glycolate, crospovidone, low-substituted hyprolose, pre-gelatinized starch.

9. A composition according to claim 6, wherein at least one binder is selected from the group consisting of povidone, copovidone, hypromellose, hyprolose, starch and pregelatinized starch.

10. A composition according to claim 6, wherein at least one lubricant is selected from the group consisting of stearic acid and its metal salts, glyceryl behenate, talc, sodium stearyl fumarate and macrogol.

11. A composition according to any one of claims 1 to 10, comprising: (a) anhydrous moxifloxacin hydrochloride and (b) microcrystalline cellulose, mannitol, croscarmellose sodium, povidone and magnesium stearate.

12. A composition according to any one of claims 1 to 10 comprising: (i) 20-70% antibiotic from the quinolone family, (ii) 2 to 10% binder, (iii) 30-60% of diluent, (iv) 1-10% disintegrant, and (v) 0.1-5% lubricant.

13. A composition according to claim 12 comprising: 40-60% anhydrous moxifloxacin hydrochloride, 15-25% microcrystalline cellulose, 5-15% mannitol, 0.5-1.5% magnesium stearate, 3-6% croscarmellose sodium and 2-4% povidone.

14. A composition according to claim 12, comprising: 50-60% anhydrous moxifloxacin hydrochloride, 18-23% microcrystalline cellulose, 11-12% mannitol, 1.0-1.5% magnesium stearate, 4-5% croscarmellose sodium, and 2.5-3.5% povidone.

15. A composition according to any one of claims 1 to 14, wherein said composition is in the form of granules, coated tablet or capsule.

16. A composition according to claim 15, wherein said composition is in the form of a coated tablet, the coating being comprised of macrogol, hypromellose, red iron oxide and titanium dioxide.

17. A process for obtaining a solid pharmaceutical composition as defined in any one of claims 1 to 16, said process comprising the steps of: (a) mixing and homogenizing, in a granulator, the active ingredient and at least one diluent and at least one disintegrant; (b) dissolving at least one binder in an organic solvent selected from the group consisting of isopropyl alcohol, acetone, ethanol, dichloromethane or mixtures thereof; (c) granulating the dried mixture of step (a) with the solution of step (b); (d) classifying and drying, at a suitable temperature, the granules of step (c); (e) classifying the dried granulate; (f) mixing and homogenizing the granules of step (e) with an additional quantity of lubricant and disintegrant to obtain the composition in the form of granules.

18. The process according to claim 17 further comprising the steps: (g) compressing the dried granulated mixture of step (f); and (h) preparing a coating mixture using an organic solvent selected from the group consisting of isopropyl alcohol, acetone, ethanol, dichloromethane or mixtures thereof, and coating the tablets obtained in step (g) to obtain a coated tablet.

19. The process according to claim 17 comprising the step of (i) encapsulating in hard gelatin capsules, the granulated dried mixture of step (f) to obtain capsules.
